# EUROPEAN PATENT SPECIFICATION

(11) **EP 1 885 353 B1**
(45) Date of publication and mention of the grant of the patent: **07.01.2015**
(21) Application number: 06751197.2
(22) Date of filing: 24.04.2006
(51) Int. Cl.: A61K 45/06, A61K 31/045, A61K 36/185, A61K 31/355

(54) **USE OF VITAMIN E TOCOTRIENOLS FOR THE INHIBITION OF INTRACELLULARLY OBLIGATE PATHOGEN CHLAMYDIA**
VERWENDUNG VON VITAMIN-E-TOCOTRIENOLEN ZUR INHIBIERUNG DES INTRAZELLULÄR OBLIGATEN PATHOGENS CHLAMYDIA
UTILISATION DE TOCOTRIENOLS DE LA VITAMINE E POUR L'INHIBITION AU NIVEAU INTRACELLULAIRE DE CHLAMYDIAE PATHOGENES OBLIGATOIRES

(30) Priority: 22.04.2005 US 673837 P; 01.03.2006 US 778432 P
(43) Date of publication of application: 13.02.2008
(73) Proprietor: Stuart, Elizabeth, Amherst, MA 01002 (US); Mueller, Anne, Sunderland, MA 01375 (US); Tan, Barrie, Amherst, MA 01002 (US)
(72) Inventor: Stuart, Elizabeth, Amherst, MA 01002 (US); Mueller, Anne, Sunderland, MA 01375 (US); Tan, Barrie, Amherst, MA 01002 (US)
(74) Representative: Vossius & Partner
(86) International application number: PCT/US2006/015398
(87) International publication number: WO 2006/116262

(56) References cited:
- STEPHENS L C ET AL: "IMPROVED RECOVERY OF VITAMIN E TREATED LAMBS THAT HAVE BEEN EXPERIMENTALLY INFECTED WITH INTRA TRACHEAL CHLAMYDIA" BRITISH VETERINARY JOURNAL, vol. 135, no. 3, 1979, pages 291-293, XP009070553 ISSN: 0007-1935
- DATABASE WPI Section Ch, Week 199317 Derwent Publications Ltd., London, GB; Class B05, AN 1993-141464 XP002393560 & SU 1 731 226 A1 (EPIDEMIOLOGY MICROBIOLOGY RES INST) 7 May 1992 (1992-05-07)
- YAMAGUCHI T: "Some antihyperlipidemic drugs might have an additional effectiveness on prevention of atherosclerosis by controlling Chlamydophila pneumoniae infection" 44TH ICAAC A MEETING OF THE AMERICAN SOCIETY FOR MICROBIOLOGY, [Online] 2 November 2004 (2004-11-02), XP002393506 Washington, DC Retrieved from the Internet: URL:http://www.asm.org/Media/index.asp?bid =31200> [retrieved on 2006-08-03]
- SHUN MING-CHIEH ET AL: "Pro-apoptotic mechanisms of action of a novel vitamin E analog (alpha-TEA) and a naturally occurring form of vitamin E (delta-tocotrienol) in MDA-MB-435 human breast cancer cells" NUTRITION AND CANCER, vol. 48, no. 1, 2004, pages 95-105, XP009070526 ISSN: 0163-5581
- PEARCE B C ET AL: "Hypocholesterolemic activity of synthetic and natural tocotrienols" JOURNAL OF MEDICINAL CHEMISTRY 1992 UNITED STATES, vol. 35, no. 20, 1992, pages 3595-3606, XP002393508 ISSN: 0022-2623
- SCHAFFER SEBASTIAN ET AL: "Tocotrienols: Constitutional effects in aging and disease" JOURNAL OF NUTRITION, vol. 135, no. 2, February 2005 (2005-02), pages 151-154, XP002393509 ISSN: 0022-3166
- HAMMERSCHLAG M R: "ADVANCES IN THE MANAGEMENT OF CHLAMYDIA PNEUMONIAE INFECTIONS" EXPERT REVIEW OF ANTI-INFECTIVE THERAPY, FUTURE DRUGS, LONDON, GB, vol. 1, no. 3, 2003, pages 493-503, XP009064049 ISSN: 1478-7210

## Description

### Other References

Azenabor AA, et al.: Chlamydia pneumoniae infected macrophages exhibit enhanced plasma membrane fluidity and show increased adherence to endothelial cells. Mol Cell Biochem 2005, 269(1-2): 69-84.
Byrne GI, et al.: Chlamydia and apoptosis: life and death decisions of an intracellular pathogen. Nat Rev Microbiol. 2004,2: 802-8.
Carabeo RA, et al.: Golgi-dependent transport of cholesterol to the Chlamydia trachomatis inclusion. Proc Natl Acad Sci USA 2003,100(11): 6771-6.
English J: Do your antioxidants suppress enough free radicals? Life Extension 2005, Feb: 22-31.
English J: Novel dietary supplement shows dramatic effects in lowering cholesterol, LDL, and triglycerides. Life Extension 2005, Nov: 28-38.
Gabel BR, et al.: Lipid raft-mediated entry is not required for Chlamydia trachomatis infection of cultured epithelial cell. Inflection and Immunity 2004, 72(12): 7367-7373.
Granato H: Cardiovascular Health. Natural Products Industry Insider 2005, Jan 3: 24-40.
Granato H: Cardiovascular Wellness. Natural Products Industry Insider 2005, Jan 6: 20-35.
Grayston JT, et al.: A new respiratory tract pathogen: Chlamydia pneumonia strain TWAR. J Infect Dis 1990, 161(4): 618-25.
Kooyenga, et al.: Antioxidants modulate the course of carotid atherosclerosis: A four- year study. Micronutrient & Health: Molecular Biological Mechanisms. Nesaretnam K, Packer L (Eds.). AOCS Press: Illinois, 2001. p. 366-375.
Ling Z, Bundey RA: Statin treatment of human vascular endothelial cells disrupts caveolae and increases nitric oxide signaling. FASEB J 2006, 20: 787.3.
Mo HB, Elson CE: Studies of the isoprenoid-mediated inhibition of mevalonate synthesis applied to cancer chemotherapy and chemoprevention. Exp Bio Med 2004, 229: 567-585.
Myers S: How to avoid a broken heart: using nutrients to control the leading risk factors of heart disease. Insider 2006, Jan 9: 22-30.
Naguib Y: Natural alternatives for maintaining healthy cholesterol. Vitamin Retailer 2004, Oct: 58-61.
Parker RA, et al.: Tocotrienols regulate cholesterol production in mammalian cells by post-transcriptional suppression of 3-hydroxy-3-methylglutaryl-coenzyme A reductase. J Biol Chem. 1993, 268: 11230-8.
Pearce BC, et al.: Hypocholesterolemic Activity of Synthetic and Natural Tocotrienols. J. Med. Chem. 1992, 35: 3595-06.
Sawayama Y, et al.: Association of Chlamydia pneumoniae antibody with the cholesterol-lowering effect of statins. Atherosclerosis 2003, 171: 281-285.
Srejic E: Keeping the ticker in tip-top shape. HSR Health Supplement Retailer 2006, March: 20- 28.
Strum S, Faloon W: Beta-Sitosterol and the aging prostate gland. Life Extension 2005, Jun: 27-31.
Stuart ES, et al.: Lipid rafts, caveolae, caveolin-1, and entry by Chlamydiae into host cells. Exp Cell Res. 2003,1:67-78.
Tan B.: Appropriate Spectrum Vitamin E and New Perspectives on Desmethyl Tocopherols and Tocotrienols. JANA 2005, 8(1): 35-42.
Yamazaki T, et al.: Biosynthesized tea polyphenols inactivate Chlamydial trachomatis in vitro. Antimicrob Agents Chemother 2005,49(6): 2501-2503.
Yamazaki T, et al.: The inhibitory effect of antihyperlipidemic drugs on the growth of Chlamydia pneumoniae in vitro. J Chemother 2006, 18(1): 107-9.

### Field of the Invention

The invention is on the use of tocotrienol to inhibit, prevent, and disrupt the developmental cell cycle and infection of *Chlamydia,* and its use to alleviate the effects of *Chlamydia-related* diseases.

### Background Of The Invention

*Chlamydia* is an obligate intracellular pathogen known to be associated with several diseases that are common today. *Chlamydia trachomatis* is the primary cause of bacterial sexually transmitted disease (STD), and can lead to ectopic pregnancies and infertility. *Chlamydia pneumoniae* causes respiratory tract infections including bronchitis, pneumonia, sinusitis, and pharyngitis. In addition, it is linked to numerous pathologies, including Alzheimer's disease, multiple sclerosis, atherosclerosis, coronary heart disease, and asthma. Three recent *Chlamydia* findings are 1) various strains of clinically important *Chlamydia* species are associated with caveolin, a molecule important to cholesterol homeostasis, 2) apoptosis (programmed cell death) is downregulated among infected cells (Byrne *et al.,* 2004), and 3) treatment of chlamydial infections with antibiotics drives the pathogens into a resistant and persistent state. Clinical persistence is an essential element of chlamydial pathogenesis, where the inability of the host to eliminate the pathogen leads to a state of chronic infectivity along with attendant tissue injury.

Delta tocotrienol is a vitamin E compound also available as dietary supplement. It has been shown to lower cholesterol in a controlled fashion, and to induce apoptosis in cancer cells, reducing tumors by as much as 70%. Since *Chlamydia* species enter cells via cholesterol-rich lipid raft domains involved in cholesterol trafficking, tocotrienols in general and hypocholesterolemic delta tocotrienol in particular will reduce infection by *Chlamydia.* Mouse macrophages (J774A1), human mammary tumor cells (MCF-7, TMX2-28), human epithelial cells (Hep-2), and human B-lymphocytes (JY) were incubated with delta tocotrienol at concentrations of 10-30 µmol/L for 6 hours prior to infection by C. *trachomatis* serovar K, a subspecies of *Chlamydia* that is the primary cause of bacteria-initiated. STD. Infections were detected by immunofluorescence staining followed by either microscopy or quantitative flow cytometric analysis. Infection levels in cells pretreated with delta-tocotrienol were decreased by >50%, with concomitant aberrant pathogen development observed with confocal microscopy. The number of large and small inclusions in the delta tocotrienol-versus-control cells was decreased by 3- and 2-fold, respectively. Flow cytometry showed that chlamydial inhibition in JY cells was at least 2-fold for an infection period of 72 hours, with a 2.6-fold maximum inhibition at 36 hours. The impact of dietary delta tocotrienol on *Chlamydia* infection in hyperlipidemic patients is being examined in a clinical study. Cholesterol-suppressive delta tocotrienol may have the potential to reduce *Chlamydia* infection in humans.

*Chlamydia* is an obligate intracellular pathogen, which means that it has to invade cells first in order to successfully survive inside the host. This infection is initiated by the so-called chlamydial elementary bodies (EBs). Once inside host cells, chlamydial replication occurs within a segregated, membrane-bound compartment called an inclusion that progressively enlarges due to metabolically active *Chlamydia* replication within the host cell.

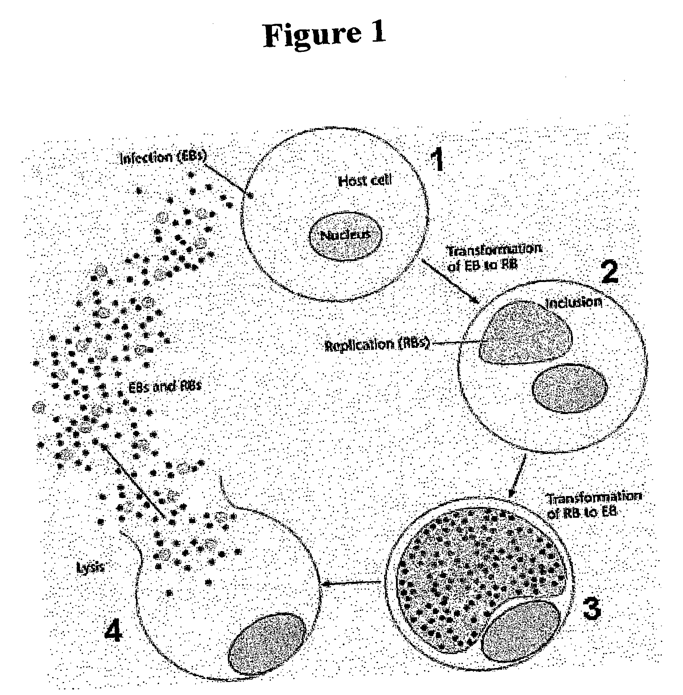

Species of the genus *Chlamydiaceae* that are frequently associated with chronic human diseases are *Chlamydiophila pneumoniae* and *Chlamydia trachomatis. C. pneumoniae* causes 5-10% of respiratory tract infections in adults and children including bronchitis, pneumonia, sinusitis, and pharyngitis. Additionally, it has been linked to chronic diseases like late onset Alzheimer's, multiple sclerosis, reactive arthritis, atherosclerosis, and asthma. Infection by *C*. *pneumoniae* has been suggested to induce autoimmunity. In multiple sclerosis (MS), 73% of the patients were shown to be *Chlamydia*-positive compared to 22% of controls. In addition, *C*. *pneumonia* is involved in atherosclerotic processes such as cellular oxidation of LDL and macrophage foam cell formation. By age 20, 50% of the population exhibits evidence of past infection by *C*. *pneumoniae* (shown by serological tests), and re-infection is common. Additionally, in a study examining a normal blood donor population for chlamydial infection levels, 25% of the population was found to be positive by immunostained blood smear. Therefore, it is possible that serological tests (e.g., ELISA) overestimate immunostaining of blood tests (e.g., actual WBCs) by ≥ two-fold. *C*. *trachomatis,* on the other hand, is the world's leading cause of preventable infectious blindness, and the most common cause of STD. In females, infection with *C*. *trachomatis* initially affects mucosal membranes and leads to continual inflammation of tissue in the genital tract, which results in scarring and eventual infertility. After invasion of cells, *Chlamydia* aggregate in vacuole-like structures called inclusions, and can escape many of the first line host defenses of the immune system. Another feature of the *Chlamydia* is that they prevent apoptosis of infected cells (Byrne, *et al.,* 2004). This effect is protective for the pathogen because it can complete a full replicative cycle within the single host cell (Figure 1). Since chlamydial inclusions inside a single infected host cell require incubation to give rise to 200-1,000 new infectious units, inhibition of host cell apoptosis by *Chlamydia* is advantageous to the pathogen and supports the establishment of a prolonged infection. Therefore, one aspect of the invention is that tocotrienols will inhibit chlamydial growth inside host cells by causing the infected host to undergo apoptosis. Another aspect of the invention is that tocotrienols will prevent the progression of the incubative cell cycle of the pathogen.

Delta tocotrienol is a vitamin E compound. Cancer studies with this compound have shown that it induces apoptosis of the tumor cells, but does not harm the surrounding healthy cells. Learning that delta tocotrienol has this ability, at least for cancer cells, triggered the idea that the compound may also up-regulate apoptosis of cells infected by *Chlamydia.* Since chlamydial inhibition of host cell apoptosis is important to the pathogen's success, such an effect by delta tocotrienol could have a profound impact as treatment against *Chlamydia.* Another delta tocotrienol characteristic of importance is its cholesterol-lowering effect (Pearce *et al.,* 1992). *Chlamydia* enters the host cell via cholesterol-rich lipid-rafts or caveolae, which are comprised of lateral assemblies of cholesterol and sphingolipids that float in the glycerophospholipid membrane, and are impaired with removal of plasma membrane cholesterol. Therefore, cells that are exposed to delta tocotrienol could remodel the lipid rafts, and thus interfere with pathogen entry into host cells directly or indirectly. Since *Chlamydia* is metabolically inactive outside the host, it is unable to survive outside the cell. A potential strategy is to inhibit cholesterol synthesis or availability in order to contain or eliminate *Chlamydia.*

In this invention, we tested delta tocotrienol's effect on chlamydial infection in numerous normal white blood cells, cancer cells, and buffy coats of *Chlamydia*-positive human blood samples.

### Summary Of The Invention

The present invention relates to the embodiments characterized in the claims. Thus, it relates to the following items:
1. A tocotrienol for use in the treatment of a *Chlamydia* infection, wherein said tocotrienol is administered for at least 3 days at a dose between 10 mg and 1000 mg per day to a mammal in need of treatment.
2. Use of a use of a tocotrienol for the preparation of a medicament for the treatment of a *Chlamydia* infection, wherein said medicament is administered for at least 3 days at a dose between 10 mg and 1000 mg per day to a mammal in need of treatment.
3. The tocotrienol of item 1 or the use of item 2, wherein the treatment inhibits, prevents or disrupts the developmental cell cycle and infection of *Chlamydia.*
4. The tocotrienol of item 1 or 3 or the use of item 2 or 3, wherein the treatment further comprises administering a geranyl geraniol.
5. The tocotrienol of any one of items 1 or 3 to 4 or the use of any one of items 2 to 4, wherein said tocotrienol is selected from the group consisting of a natural tocotrienol and a synthetic tocotrienol.
7. The tocotrienol or the use of item 5, wherein said tocotrienol is selected from the group consisting of alpha-tocotrienol, beta-tocotrienol, gamma-tocotrienol, delta-tocotrienol, desmethyl-tocotrienol, and didesmethyltocotrienol.
8. The tocotrienol or the use of item 7, wherein the tocotrienol is delta-tocotrienol.
9. The tocotrienol of any one of items 1 or 3 to 8 or the use of any one of items 2 to 8, wherein the *Chlamydia* is selected from the group consisting of *Chlamydia trachomatis, Chlamydia suis, Chlamydia muridarum, Chlamydiophila pneumoniae, Chlamydiophila psittaci, Chlamydiophila pecorum, Chlamydiophila abortis, Chlamydiophila felis,* and *Chlamydiophila caviae.*
10. The tocotrienol of any one of items 1 or 3 to 9 or the use of any one of items 2 to 9, wherein the tocotrienol stunts the growth and inhibits *Chlamydia* maturation of reticulate bodies into elementary bodies, preventing its progression and development.
11. The tocotrienol of any one of items 1 or 3 to 10 or the use of any one of items 2 to 10, wherein the tocotrienol reduces chlamydial infection of a white blood cell.
12. The tocotrienol or the use of item 11, wherein the white cell is selected from the group consisting of neutrophils, monocytes, lymphocytes, eosinophils, and basophils.
13. The tocotrienol of any one of items 1 or 3 to 12 or the use of any one of items 2 to 12, wherein the mode of application of the tocotrienol is selected from the group consisting of aerosol spray, oral ingestion, creams, douches, lotions, and eye drops.
14. The tocotrienol or the use of item 13, comprising administering a dose of tocotrienol between 20 mg and 500 mg per day, or between 50 mg and 150 mg per day.
15. A tocotrienol for use in the treatment of a *Chlamydia* infection, wherein said tocotrienol is administered in combination with at least one agent selected from the group consisting of a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.
16. Use of tocotrienol for the preparation of a medicament for the treatment of a *Chlamydia* infection, wherein said tocotrienol is administered in combination with at least one agent selected from the group consisting of a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.
17. The tocotrienol of item 15 or the use of item 16, wherein the agent is citrus bioflavonoid.
18. A tocotrienol for use in the treatment of a *Chlamydia* infection, wherein tocotrienol is administered for at least 3 days to a mammal or avian, wherein said mammal or avian suffers from a *Chlamydia*-associated disease selected from the group consisting of cardiovascular disease, hypertension, atherosclerosis, COX-I- and COX-II-induced inflammation, a sexually transmitted disease, a genital tract infection, arthritis, prediabetes, metabolic syndrome, diabetes, polycystic ovarian syndrome (PCOS), a respiratory tract infection, pneumonia, an ocular infection, a neurological disease, Alzheimer's disease, and multiple sclerosis.
19. Use of tocotrienol for the preparation of a medicament for the treatment of a *Chlamydia* infection, wherein tocotrienol is administered for at least 3 days to a mammal or avian, wherein said mammal or avian suffers from a *Chlamydia-*associated disease selected from the group consisting of cardiovascular disease, hypertension, atherosclerosis, COX-I- and COX-II-induced inflammation, a sexually transmitted disease, a genital tract infection, arthritis, prediabetes, metabolic syndrome, diabetes, polycystic ovarian syndrome (PCOS), a respiratory tract infection, pneumonia, an ocular infection, a neurological disease, Alzheimer's disease, and multiple sclerosis.
20. A tocotrienol for use in improving immunity to *Chlamydia,* wherein said tocotrienol potentiates an antigen-presenting dendritic cell.
21. Use of tocotrienol for the manufacture of a medicament for improving immunity to *Chlamydia,* wherein said tocotrienol potentiates an antigen-presenting dendritic cell.

Moreover, uses of Vitamin E are disclosed to inhibit and prevent infection by *Chlamydia.* Additionally, there is disclosed the mechanism of action by Vitamin E, especially delta-tocotrienol, to interrupt the infection process of a *Chlamydia.*

There is disclosed a method of using Vitamin E tocochromanol to inhibit, prevent, and disrupt the developmental cell cycle and infection of *Chlamydia.* There is disclosed a method of using tocotrienol to inhibit, prevent, and disrupt the developmental cell cycle and/infection of *Chlamydia.* There is also disclosed a method of using delta-tocotrienol to inhibit, prevent, and disrupt the developmental cell cycle and infection of *Chlamydia.*

There is also disclosed a method of using Vitamin E tocochromanol to alleviate the effects of *Chlamydia*-related diseases.

There is disclosed a method of using tocotrienol to alleviate the effects of *Chlamydia*-related diseases. There is also disclosed a method of using delta-tocotrienol to alleviate the effects of *Chlamydia*-related diseases.

There is also disclosed a method of using Vitamin. E. tocochromanol to reduce the quantity of chlamydial inclusions.

There is disclosed a method of using tocotrienol to reduce the quantity of chlamydial inclusions. There is disclosed a method of using delta-tocotrienol to reduce the quantity of chlamydial inclusions.

There is disclosed a method of using Vitamin E tocochromanol to impede and suppress the initial infection by chlamydial EBs of host cells.

There is disclosed a method of using tocotrienol to impede and suppress the initial infection by chlamydial EBs of host cells.

There is disclosed a method of using delta-tocotrienol to impede and suppress the initial infection by chlamydial EBs of host cells.

There is disclosed a method of using 5 µmol/L delta-tocotrienol to inhibit chlamydial development within the host cell.

There is disclosed a method of using 10 µmol/L delta-tocotrienol to inhibit chlamydial development within the host cell. There is disclosed a method of using 20 µmol/L delta-tocotrienol to inhibit chlamydial development within the host cell. There is disclosed a method of using 30 µmol/L delta-tocotrienol to inhibit chlamydial development within the host cell. There is disclosed a method of using 40 µmol/L delta-tocotrienol to inhibit chlamydial development within the host cell.

There is disclosed a method of using delta-tocotrienol several times to reduce the level of infection by *Chlamydia.* There is disclosed a method of using delta-tocotrienol 3 to 10 times to reduce the level of infection by *Chlamydia.* There is disclosed a method of using delta-tocotrienol more than 10 times to reduce the level of infection by *Chlamydia.* There is disclosed a method of using delta-tocotrienol repeatedly to reduce the level of infection by *Chlamydia*

- There is disclosed a method of using delta-tocotrienol several times to inhibit re-infection of the host cell by chlamydial EBs.

There is disclosed a method of using delta-tocotrienol 3 to 10 times to inhibit re-infection of the host cell by chlamydial EBs. There is disclosed a method of using delta-tocotrienol more than 10 times to inhibit re-infection of the host cell by chlamydial EBs. There is disclosed a method of using delta-tocotrienol repeatedly to inhibit re-infection of the host cell by chlamydial EBs.

There is disclosed a method of using tocotrienol to inhibit inclusion formation by 5%. There is disclosed a method of using tocotrienol to inhibit inclusion formation by 10%.

There is disclosed a method of using tocotrienol to inhibit inclusion formation by 15%. There is disclosed a method of using tocotrienol to inhibit inclusion formation by 20%. There is disclosed a method of using tocotrienol to inhibit inclusion formation by 25%. There is disclosed a method of using tocotrienol to inhibit inclusion formation by 30%. There is disclosed a method of using tocotrienol to inhibit inclusion formation by 40%.

There is disclosed a method of using tocotrienol to inhibit inclusion formation by 45%. There is disclosed a method of using tocotrienol to inhibit inclusion formation by 50%.

There is disclosed a method of using tocotrienol to inhibit inclusion formation by 60%. There is disclosed a method of using tocotrienol to inhibit inclusion formation by 75%.

There is disclosed a method of using tocotrienol to inhibit inclusion formation by 90%.

There is disclosed a method of using Vitamin E tocochromanol. There is disclosed a method of using tocotrienol for at least one day. There is disclosed a method of using tocotrienol for at least three days. There is disclosed a method of using tocotrienol for more than three days.

There is disclosed a method of using tocotrienol effective in humans against *Chlamydia* as represented in circulating WBCs, including neutrophils, monocytes, lymphocytes, eosinophils, and basophils.

There is disclosed a method of using tocotrienol to reduce the cholesterol level and chlamydial infection in hyperlipidemic patients, and rendered the chlamydial infection status negative.

There is disclosed a method of using an agent that restricts cholesterol/lipids to restrict *Chlamydia* infection and growth.

There is disclosed a method of using tocotrienol to reduce *Chlamydia* infections in cancer patients.

There is disclosed a method of using tocotrienol to ward off pathogenic infections including *Chlamydia* via activation of antigen-presenting dendritic cells and T-lymphocytes.

There is disclosed a method of using tocotrienol to resist opportunistic chlamydial infection of hypertensive patients, and thereby reduce the patient's blood pressure.

There is disclosed a method of using tocotrienol to reduce cardiovascular risk factors associated with metabolic syndrome, and thereby reduce the risk of diabetes and concomitant or subsequent infection by *Chlamydia.*

There is disclosed a method of application of tocotrienol by aerosol sprays (to reach respiratory tract airways), oral ingestion via softgels, tablets or capsules (to reach vascular-organistic systems), topical creams, douches, and lotions (to reach genital sites), and topical liquid drops (to reach ocular sites).

There is disclosed a method of application of tocotrienol by oral ingestion via softgels, tablets or capsules, and topical creams, douches, and lotions. There is disclosed a method of application of tocotrienol by oral ingestion via softgels, tablets or capsules. There is disclosed a method of administering delta tocotrienol in a range from 10 to 1000 mg per day. There is disclosed a method of administering delta tocotrienol in a range from 20 to 500 mg per day.

There is disclosed a method of administering delta tocotrienol in a range from 50 to 150 mg per day. There is disclosed a method of treatment with delta tocotrienol of administering daily for one month.

There is disclosed a method of treatment with delta tocotrienol of administering daily for six months.

There is disclosed a method of treatment with delta tocotrienol of administering daily for one year. There is disclosed a method of treatment with delta tocotrienol of administering daily until infection and inflammation due to *Chlamydia* is cleared.

There is disclosed a method of using tocotrienol for respiratory tract infections by using an aerosol spray at a dosage of 1 spray per day.

There is disclosed a method of using tocotrienol for respiratory tract infections by using an aerosol spray at a dosage of 2 sprays per day.

There is disclosed a method of using tocotrienol for respiratory tract infections by using an aerosol spray at a dosage of 4 sprays per day.

There is disclosed a method of using tocotrienol for genital tract infections by using an aerosol spray at a dosage of 1 application per day.

There is disclosed a method of using tocotrienol for genital tract infections by using an aerosol spray at a dosage of 2 applications per day.

There is disclosed a method of using tocotrienol for genital tract infections by using an aerosol spray at a dosage of 4 applications per day.

There is disclosed a method of using tocotrienol to reduce inflammation of vascular-organistic systems due to chlamydial infection by oral ingestion of 1 dose per day. There is disclosed a method of using tocotrienol to reduce inflammation of vascular-organistic systems due to chlamydial infection by oral ingestion of 2 doses per day. There is disclosed a method of using tocotrienol to reduce inflammation of vascular-organistic systems due to chlamydial infection by oral ingestion of 4 doses per day.

There is disclosed a method of using tocotrienol to reduce ocular infections and conjunctivitis due,to chlamydial infection by applying liquid eye drops with 1 application per day. There is disclosed a method of using tocotrienol to reduce ocular infections and conjunctivitis due to chlamydial infection by applying liquid eye drops with 2 applications per day.

There is disclosed a method of using tocotrienol to reduce ocular infections and conjunctivitis due to chlamydial infection by applying liquid eye drops with 4 applications per day.

There is disclosed a method of using tocotrienol where the delta-to-gamma ratio of tocotrienols is 1:100 to 100:1. There is disclosed a method of using tocotrienol where the delta-to-gamma ratio of tocotrienols is 1:25 to 25:1. There is disclosed a method of using tocotrienol where the delta-to-gamma ratio of tocotrienols is 1:10 to 10:1.

There is disclosed a method of using tocotrienol where the delta-to-gamma ratio of tocotrienols is 1:5 to 5:1.

There is disclosed a method of using tocotrienol where the delta-to-gamma ratio of tocotrienols is 1:1.

There is disclosed a method of using tocotrienol comprising a mixture of annatto extract and a natural extract that is an appropriate spectrum. There is disclosed a method of using tocotrienol where more than 50% of the tocotrienols are delta-T3 and gamma-T3.

There is disclosed a method of using tocotrienol where more than 50% of the tocotrienols are delta-T3. There is disclosed a method of using tocotrienol where it is tocopherol-free.

There is disclosed a method of using tocotrienol where the C5 unsubstituted tocotrienols are >60%, and tocopherols are <15%.

There is disclosed a method of using tocotrienol where the C5 unsubstituted tocotrienols are >70% C5 unsubstituted tocotrienols and<10% tocopherols.

There is disclosed a method of using tocotrienol where the C5 unsubstituted tocotrienols are >80% C5 unsubstituted tocotrienols and <5% tocopherols.

There is disclosed a method of using tocotrienol where the method of using tocotrienol is tocopherol-free with >98% tocotrienols, and tocotrienols are predominantly delta T3 and gamma-T3. There is disclosed a method of using tocotrienol where the tocotrienol is tocopherol-free with >98% tocotrienols and tocotrienols are predominantly delta-T3.

There is disclosed a method of using tocotrienol comprising annatto extract where C5 unsubstituted tocols inhibit surface chemotactic bioactive materials (CBM). There is disclosed a method of using tocotrienol comprising annatto extract where annatto C5 unsubstituted T3 inhibit surface chemotactic bioactive materials. There is disclosed a method of using tocotrienol comprising annatto extract where annatto C5 unsubstituted T3 inhibit surface chemotactic bioactive materials and prevent the tether or adhesion of circulating monocytes and leucocytes onto stationary endothelia.

There is disclosed a method of using tocotrienol comprising annatto extract where annatto C5 unsubstituted T3 inhibit surface chemotactic bioactive materials and prevent the tether or adhesion of circulating monocytes and leucocytes onto stationary endothelia that cause the loss of vasculature integrity.

There is disclosed a method of using tocotrienol comprising annatto extract where annatto C5 unsubstituted T3 inhibit surface chemotactic bioactive materials and prevent the tether or adhesion of circulating monocytes and leucocytes onto stationary endothelia that cause the loss of vasculature integrity, and prevent micro- and macro-vascular diseases, and atherosclerosis. There is disclosed a method of using tocotrienol comprising annatto extract where annatto C5 unsubstituted T3 inhibit CBM and prevent pathological events selected from the group consisting of chemotaxis, vasoconstriction, hypercoagulation, glycoxidation and oxidized LDL by via HDL elevation.

There is disclosed a method of using tocotrienol where the tocotrienol is combined with other nutrients. There is disclosed a method of using tocotrienol were the tocotrienol is combined with other nutrients, and the tocotrienol contains geranyl geraniol. There is disclosed a method of using tocotrienol where the nutrient is selected from the group consisting of phytosterols, oryzanols, policosanols, pantethine, red yeast rice (Monascus), oat bran, garlic, gugul lipids, chitosan, soy protein (e.g., oligo- and polypeptides, hydrolysates), CoQ10, carnitine, magnesium, chromium, potassium, calcium, D-tyrosine, fibers (insoluble and soluble types, including beta-glucans), omega-3s (DHAs and EPAs, ALAs), and lecithin.

There is disclosed a method of using geranyl geraniols and tocotrienols and it increases the de novo biosyntheses of all subsequent intermediate isoprenoid pool and distal products.

There is disclosed a method of using tocotrienol containing geranyl geraniols that anabolically increases the endogenous de novo synthesis of CoQ10 via geranyl geraniols elongation/prenylation of side chain and conversely CoQ10 catabolically increases the endogenous de novo synthesis of geranyl geraniols via CoQ 10 beta-oxidation.

There is disclosed a method of supplementation, comprising the administering of a tocotrienol or a tocotrienol and geranyl geraniol, and reducing Chlamydia-induced blindness.

### Detailed Description of the Preferred Embodiment

It is disclosed that the method administers a tocotrienol, where the tocotrienol contains delta tocotrienol and gamma-tocotrienol, and where the delta-to-gamma ratio of tocotrienols is 1:100 to 100:1. It is disclosed that the method administers an tocotrienol, where the tocotrienol contains delta-tocotrienol and gamma-tocotrienol, and where the delta-to-gamma ratio of tocotrienols is 1:25 to 25:1. It is disclosed that the method administers an tocotrienol, where the tocotrienol contains delta-tocotrienol and gamma tocotrienol, and where the delta-to-gamma ratio of tocotrienols is 1:10 to 10:1. It is disclosed that the method administers a tocotrienol, where the tocotrienol contains delta-tocotrienol and gamma-tocotrienol, and where the delta-to-gamma ratio of tocotrienols is 1:5 to 5:1. It is disclosed that the method administers an tocotrienol, where the tocotrienol contains delta-tocotrienol and gamma-tocotrienol, and where the delta-to-gamma ratio of tocotrienols is 1:1.

It is disclosed that a method administers a tocotrienol, where the method of using tocotrienol is a mixture of an annatto extract and a natural extract, and where the mixture has standardized low levels of tocopherols. It is disclosed that the method administers a tocotrienol, where the method of using tocotrienol is a mixture of an annatto extract and a natural extract, and where the standardized level of tocopherols is ≤ 50%.

It is disclosed that the method administers a tocotrienol, where the method of using tocotrienol, is a mixture of an annatto extract and a natural extract, and where the standardized level of tocopherols is ≤ 20%. It is disclosed that the method administers a tocotrienol, where the method of using tocotrienol is a mixture of an annatto extract and a natural extract, and where the standardized level of tocopherols is ≤ 10%.

It is disclosed that the method administers a tocotrienol, where the method of using tocotrienol is a mixture of an annatto extract and a natural extract, and where the standardized level of tocopherols is ≤ 1%. It is disclosed that the method administers a tocotrienols, where the natural extract is selected from the group consisting of a vegetable oil of rice bran, palm, cranberry seed, and litchi seed.

It is disclosed that a method administers a mixture of annatto extract and a natural extract that is an appropriate spectrum. It is disclosed that the method administers a mixture of annatto extract and a natural extract, and more than 50% of the tocotrienols are delta-T3 and gamma-T3. It is disclosed that the method administers a mixture of annatto extract and a natural extract, and more than 50% of the tocotrienols are delta-T3. It is disclosed that the method administers a mixture of annatto extract and a natural extract, and it is tocopherol-free.

It is disclosed that a method administers an >60% C5 unsubstituted tocotrienols and <15% tocopherols. It is disclosed that a method administers an >70% C5 unsubstituted tocotrienols and <10% tocopherols. It is disclosed that a method administers an >80% C5 unsubstituted tocotrienols and <5% tocopherols.

It is disclosed that a method administers an annatto extract and the method of using tocotrienol is tocopherol-free with >98% tocotrienols, and tocotrienols are predominantly delta-T3 and gamma-T3. It is disclosed that the method administers an annatto extract and is tocopherol-free with >98% tocotrienols and tocotrienols are predominantly delta-T3.

It is disclosed that a method administers an C5 unsubstituted tocols inhibit surface chemotactic bioactive materials. It is disclosed that a method administers an C5 unsubstituted tocols, where the C5 unsubstituted tocols are C5 unsubstituted T3 and the C5 unsubstituted T3 inhibit surface chemotactic bioactive materials. It is disclosed that a method administers an C5 unsubstituted tocols, where the C5 unsubstituted tocols are C5 unsubstituted T3 and the C5 unsubstituted T3 inhibit surface chemotactic bioactive materials and prevent the tether or adhesion of circulating monocytes and leucocytes onto stationary endothelia. It is disclosed that a method administers an C5 unsubstituted tocols, where the C5 unsubstituted tocols are C5 unsubstituted T3 and the C5 unsubstituted T3 inhibit surface chemotactic bioactive materials and prevent the tether or adhesion of circulating monocytes and leucocytes onto stationary endothelia that cause the loss of vasculature integrity. It is disclosed that a method administers an C5 unsubstituted tocols, where the C5 unsubstituted tocols are C5 unsubstituted T3 and the C5 unsubstituted T3 inhibit surface chemotactic bioactive materials and prevent the tether or adhesion of circulating monocytes and leucocytes onto stationary endothelia that cause the loss of vasculature integrity, and prevent micro- and macro-vascular diseases, and atherosclerosis. It is disclosed that a method administers an C5 unsubstituted tocols, where the C5 unsubstituted tocols are C5 unsubstituted T3 and the C5 unsubstituted T3 inhibit surface chemotactic bioactive materials and prevent pathological events selected from the group consisting of chemotaxis, vasoconstriction, hypercoagulation, glycoxidation and oxidized LDL by via HDL elevation.

It is disclosed that a method administers an annatto extract and the annatto extract is combined with other nutrients. It is disclosed that a method administers an annatto extract and the annatto extract is combined with other nutrients, and the annatto extract contains tocotrienol and geranyl geraniol. It is disclosed that a method administers an annatto extracts and where the nutrient is selected from the group consisting of phytosterols, oryzanols, policosanols, pantethine, red yeast rice (Monascus), oat bran, garlic, gugul lipids, chitosan, soy protein (e.g., oligo- and poly-peptides, hydrolysates), CoQ10, carnitine, magnesium, chromium, potassium, calcium, D-tyrosine, fibers (insoluble and soluble types, including beta-glucans), omega-3s (DHAs and EPAs, ALAs), and lecithin. It is disclosed that a method administers an annatto extract and a nutrient, and the nutrient is selected from the group consisting of banaba extract (e.g., corosolic acid), lipoic acids (all isomeric forms), chromium, and the B vitamins including niacin.

It is disclosed that a method administers an geranyl geraniols and tocopherol-free C-5 unsubstituted tocotrienols. It is disclosed that the method administers an geranyl geraniols, tocopherol-free C-5 unsubstituted tocotrienols, and inactive and/or active ingredients.

There is also disclosed a method of using tocotrienol containing geranyl geraniols treats a disease of the nervous system. It is disclosed the method of using tocotrienol containing geranyl geraniols treats a disease of the nervous system, where the disease is selected from the group consisting of chronic Alzheimer's, Parkinson's, Familial Dysautonomia, Muscular Sclerosis, and Muscular Atrophy.

In the present context there is also disclosed the following with reference to the numbered paragraphs below:
1. A method of treating an infection by *Chlamydia,* comprising administering a Vitamin E tocochromanol to a mammal in need of treatment.
2. The method of paragraph 1, where the treatment inhibits the developmental cell cycle and infection of *Chlamydia.*
3. The method of paragraph 1, where the treatment prevents the developmental cell cycle and infection of *Chlamydia.*
4. The method of paragraph 1, where the treatment disrupts the developmental cell cycle and infection of *Chlamydia.*
5. The method of paragraph 1, further comprising administering a geranyl geraniol.
6. The method of paragraph 1, where the mammal has a condition selected from the group consisting of elevated intracellular calcium, increased caveolae expression, increased vasoconstriction, hypertension and primary pulmonary hypertension.
7. The method of paragraph 1, where the Vitamin E tocochromanol is selected from the group consisting of a natural tocopherol, a synthetic tocopherol, a natural tocotrienol and a synthetic tocotrienol.
8. The method of paragraph 7, where the tocotrienol is an isomer of a tocotrienol.
9. The method of paragraph 8, where the isomer of tocotrienol is selected from the group consisting of alpha, beta, gamma, delta, desmethyl, and didesmethyl.
10. The method of paragraph 7, where the tocopherol is an isomer of a tocopherol.
11. The method of paragraph 10, where the isomer of tocopherol is selected from the group consisting of alpha, beta, gamma, delta, desmethyl, and didesmethyl.
12. The method of paragraph 1, where the *Chlamydia* is selected from the group consisting of *Chlamydia trachomatis, Chlamydia suis, Chlamydia muridarum, Chlamydiophila pneumoniae, Chlamydiophila psittaci, Chlamydiophila pecorum, Chlamydiophila abortis, Chlamydiophila felis,* and *Chlamydiophila caviae.*
13. The method of paragraph 1, where the tocotrienol stunts the growth and inhibits *Chlamydia* maturation of reticulate bodies into elementary bodies, preventing its progression and development.
14. The method of paragraph 1, where the tocotrienol reduces chlamydial infection of a white blood cell.
15. The method of paragraph 14, where the white cell is selected from the group consisting of neutrophils, monocytes, lymphocytes, eosinophils, and basophils.
16. The method of paragraph 7, where the tocotrienol lowers cholesterol in hypercholesterolemic patients to inhibit chlamydial infection.
17. A method of treating an infection by *Chlamydia,* comprising administering an agent that restricts cholesterol.
18. The method of paragraph 17, where the agent that restricts cholesterol is selected from the group consisting of a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.
19. The method of paragraph 18, where the bioflavonoid is selected from the group consisting of citrus bioflavonoid and polymethoxylated flavone.
20. The method of paragraph 1, where mode of application of the Vitamin E tocochromanol is selected from the group consisting of aerosol spray, oral ingestion, creams, douches, lotions, and eye drops.
21. The method of 20, comprising administering a dose of tocotrienol between 10 mg and 1000 mg per day.
22. The method of 21, comprising administering a dose of tocotrienol between 20 mg and 500 mg per day.
23. The method of 22, comprising administering a dose of tocotrienol between 50 mg and 150 mg per day.
24. A method of treating an infection by *Chlamydia,* comprising administering a combination of a tocotrienol and at least one agent selected from the group consisting of a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.
25. A method of treating an infection by *Chlamydia,* comprising administering a combination of a polymethoxylatyed flavone and at least one agent selected from the group consisting of a tocotrienol, a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.
26. A method of treating *Chlamydia*-associated diseases, comprising administering a hypocholesterolemic agent to a mammal or avian to treat a *Chlamydia*-associated disease selected from the group consisting of cardiovascular disease, hypertension, atherosclerosis, COX-I- and COX-II-induced inflammation, a sexually transmitted disease, a genital tract infection, arthritis, prediabetes, metabolic syndrome, diabetes, polycystic ovarian syndrome (PCOS), a respiratory tract infection, pneumonia, an ocular infection, a neurological disease, Alzheimer's disease, and multiple sclerosis.
27. The method of Paragraph 26, where the hypocholesterolemic agent is a tocotrienol.
28. The method of Paragraph 27, where tocotrienol inhibits intracellular calcium, [Ca²⁺], and caveolae expression, and thereby reduces vasoconstriction and primary pulmonary hypertension (PPH), and therefore inhibits chlamydial infection associated with hypertension.
29. A method to improve immunity to *Chlamydia,* comprising administering a tocotrienol to a mammal to potentiate an antigen-presenting dendritic cell and improve immunity against *Chlamydia.*
30. A method to inhibit progression of a cancer, comprising administering a tocotrienol to a mammal to potentiate an antigen-presenting dendritic cell and inhibit progression of a cancer.

### Other Embodiments

It is to be understood that while the invention has been described in conjunction with the detailed description thereof, the foregoing description is intended to illustrate the invention, which is defined by the scope of the appended claims. For example, although the above description relates to human cells, various aspects of the invention might also be applied to cells from other animals (e.g., mammals, avians, fish, crustaceans, and domestic and farm animals) by making appropriate modifications to the described methods.

### Brief Description Of The Several Views Of The Drawing

Figure 1 illustrates the Chlamydial Developmental Cycle. In step (1), elementary bodies (EBs) infect host cells by a process similar to receptor-mediated endocytosis, and form a vacuole-like structure called an inclusion (2). The EBs then transform into non-infectious reticulate bodies (RBs), in which state they replicate and push the nucleus to the side of the cell (2→3). The inclusion enlarges, the RBs transform back to EBs (3), and the inclusion eventually lyses the cell (4). The freed infectious EBs are now able to re-infect surrounding host cells.

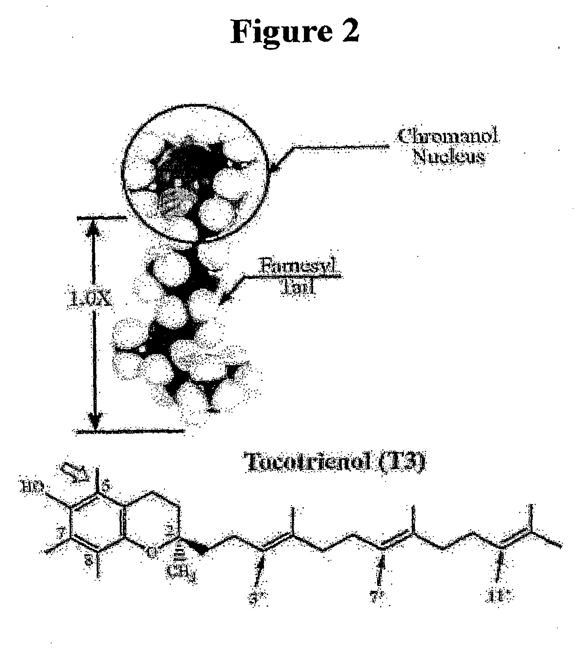

Figure 2 illustrates the Molecular and Chemical Structure of Delta Tocotrienol. Delta tocotrienol is a Vitamin E compound with a chromanol nucleus (site of antioxidant activity, typical for all Vitamin E compounds), and an isoprenoid tail (farnesyl tail). The famesylated tail downregulates the rate-limiting cholesterol biosynthesis enzyme, 3-hydroxy-3-methylglutaryl coenzyme A (HMG-CoA) reductase (Parker *et al.,* 1993). This cholesterol inhibition by delta tocotrienol is unique to famesylated Vitamin E compounds. Among the known isomers of tocotrienol are alpha, beta, gamma, and delta tocotrienol. The potency of cholesterol inhibition by these isomers is delta>gamma>beta>alpha tocotrienol. Apparently, desmethyl tocotrienols are more active, especially in the absence of a methyl group at C5 on the benzene ring (see arrow). Delta tocotrienol is monomethylated at position 8 of the benzene ring, making it the least substituted, and therefore the most potent isomer of the four common tocotrienol compounds (Tan, 2005).

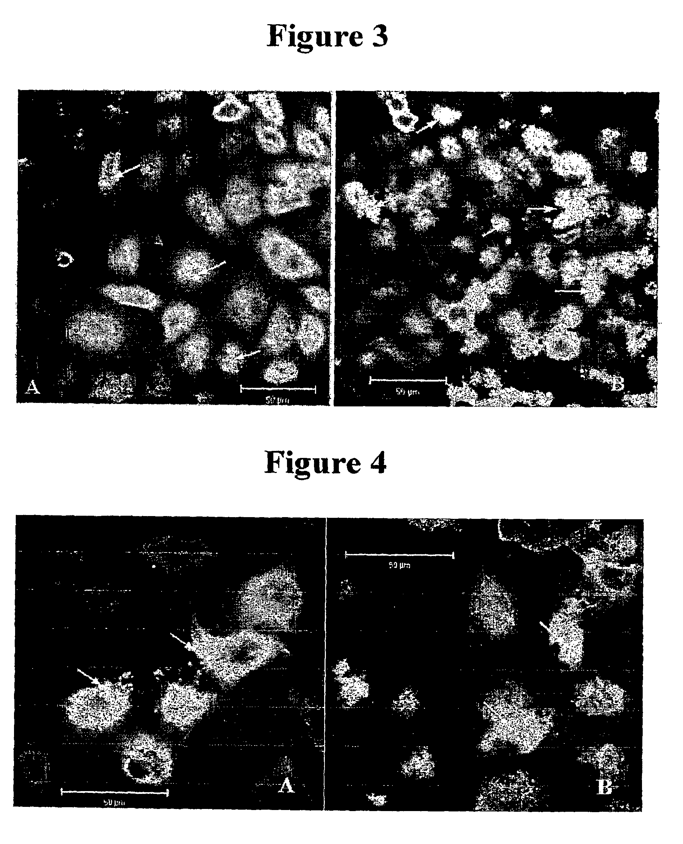

Figure 3 illustrates *Chlamydia*-infected mouse macrophage cells treated with delta tocotrienol (400X magnification). Mouse macrophages (J774A.1 cells) were pretreated with a 30 µmol/L concentration of delta tocotrienol in culture for 4 hours and infected with *C*. *trachomatis* SVK for 48 hours. Arrows indicate inclusions. Cells were viewed and digitally documented using a Zeiss LSM 510 Meta Confocal System at a magnification of 400X. White arrows point out characteristic inclusions (Scale indicates 50 µm).
Figure 4 illustrates *Chlamydia*-infected mouse macrophage cells treated with delta tocotrienol (630X magnification). White arrows point to representative cells. Note numerous very small, unfused inclusions in A (Scale indicates 50 µm).

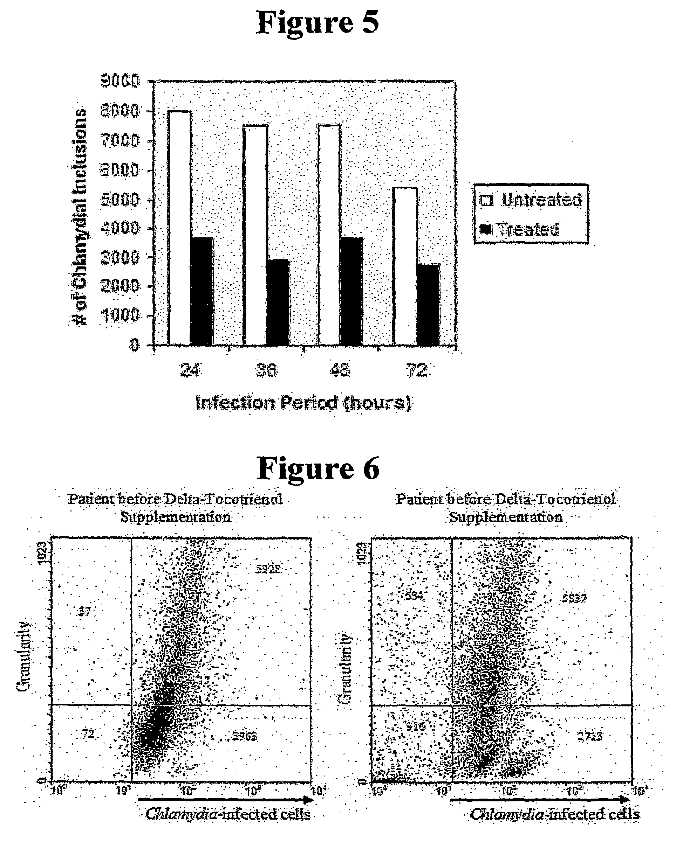

Figure 5 illustrates flow cytometry on *Chlamydia*-infected human B-lymphocytes treated with delta tocotrienol. Human B-lymphocyte (JY) cells were treated with a 30 µmol/L concentration of delta tocotrienol in culture for 4 hours, infected with *C*. *trachomatis* SVK for 24-72 hours. Immunofluorescent *Chlamydia*-positive cells were detected by flow cytometry.
Figure 6 illustrates detection and quantification of *Chlamydia*-infected white blood cells in hyperlipidemic patients with delta-tocotrienol supplementation. Buffy coats of hyperlipidemic patients were immunostained, and for each sample, aliquots of 10,000 cells were assessed. A dotplot of a hyperlipidemic patient prior to supplementation with delta-tocotrienol is shown in the left panel, and a dotplot of a hyperlipidemic patient after 2 months of delta-tocotrienol supplementation (100 mg/day) is shown in the right panel.

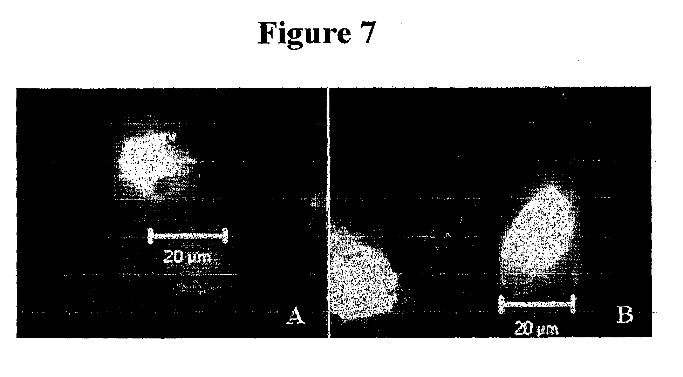

Figure 7 illustrates *Chlamydia*-infected human mammary tumor cells treated with delta tocotrienol (630X magnification). Human mammary tumor cells (TMX2-28) were infected with *Chlamydia* for 72 hours and treated with a 15 µmol/L concentration of delta tocotrienol (Figure 7A). Controls were left untreated (Figure 7B).

### Detailed Description Of The Invention

The developmental cycle of *Chlamydia* consists of the infectious, but metabolically inactive elementary body (EB) that will initiate cell entry, and the metabolically active, but non-infectious reticulate body (RB), which replicates within the cell and differentiates back to EBs prior to release from the infected cell [Figure 1].

Microbes usurp normal host cell endocytic pathways to gain entry. Several infectious agents, including viruses and intracellular parasites, were found to enter host cells via caveolae or rafts. Many mechanisms for the entry of *Chlamydia* into host cells are proposed. These include the passage-like caveolae, cavity-like clathrin, and/or other pathways. Since only the EB form of *Chlamydia* is infectious and the EB at 300 nm is about 3-fold larger than either caveolae or clathrin (both ca. 100 nm), it seems unlikely that *Chlamydia* would enter host cells via these mechanisms. *Chlamydia* entry into host cells and subsequent infection requires lipid rafts, especially those that are rich in cholesterol content. Severe sequestration of cholesterol by cholesterol precipitation/chelation (Stuart *et al.,* 2003), as well as, inhibition of cholesterol synthesis using antihyperlipidemic drugs (Yamazaki *et al.,* 2006) interferes with host cell endocytosis of *Chlamydial.* Therefore, *Chlamydia* is more likely to enter cells via lipid rafts than some form of caveolar enclosure (invagination). In addition, statins were found to disrupt caveolae in human vascular endothelial cells, thus providing an alternate route of Chlamydia inhibition (Ling and Bundey, 2006), hitherto unknown. Such route of Chlamydia inhibition may further be supported by reduction of cholesterol with statin. The raft-mediated endocytosis may utilize a zipper-type process, whereby the lipids fuse or coalesce onto the EB to facilitate entry. Such fusion of cholesterol materials onto the EB is plausible, since *Chlamydia* cannot synthesize its own lipids, and is known to obtain cholesterol from its host. This cholesterol from the host may be from *de novo* synthesis in the host cell or from the systemic circulation *ex vivo* where it was transported from the liver. *Chlamydia* obtains cholesterol preferentially from extracellular sources by trafficking from the Golgi apparatus, which is then found in large amounts in the chlamydial inclusion membrane (Carabeo *et, al.*, 2003). *De novo*-synthesized cholesterol is mediated via lipid-rich transport intermediates by energy- and temperature-dependent transport to the plasma membrane.

In a 2-year clinical study on hyperlipidemic patients positive for *C. pneumoniae,* the cholesterol-lowering drug pravastatin did not induce a significant decrease in serum total- and LDL-cholesterol (1-2% drop), but reduced carotid atherosclerosis by 12%, significantly (Sawayama *et al.,* 2003). This study is instructive in that if these patients were not *Chlamydia*-positive, cholesterol levels might have decreased significantly by administration of pravastatin, and carotid atherosclerosis might have been reduced by more than 12%. Another study found that *Chlamydia*-infected cells showed an enhanced uptake of LDL in macrophages, thereby contributing to foam cell and eventual atherogenic lesion formation. Put together, these studies imply that a relationship exists between *Chlamydial* infection, cholesterol, and carotid atherosclerosis. Earlier pathological studies have shown a positive association between prior or current infection with *C. pneumoniae* and coronary artery disease (Grayston *et al.,* 1990). Interestingly, in a 4-year study, tocotrienol caused a regression of human carotid atherosclerosis without a significant cholesterol drop (Kooyenga *et al.,* 2001).

Tocotrienols [Figure 2] belong to the same group of vitamin E as tocopherols, and their ring structure gives these compounds antioxidant properties. Tocotrienol specifically inhibits *de novo* synthesis of cholesterol via the HMG-CoA reductase pathway. It is expected that the tocotrienol, while lowering systemic LDL, will compromise the constitution of the cholesterol-rich lipid rafts in host cell outer membranes. Importantly, tocotrienol inhibits monocyte-endothelial cell adhesion, which in turn aids a more uniform distribution in the membrane bilayer, and greatly reduces the risk of developing atherosclerotic lesions. Therefore, it is further expected that tocotrienol inhibits the infection of pathogen to host by adhesion of *Chlamydia* to the host.

Once chlamydial entry into host cells has occurred, the bacteria have an ongoing requirement for cholesterol to continue pathogen development. This cholesterol is specifically derived from the membrane of host cells (Azenabor, 2005), and it has been shown that this process is mediated via the Golgi organelles (Carabeo *et al.,* 2003). The depletion of cholesterol from host cell membrane occurs because the *Chlamydial* inclusion hijacks cholesterol the host cell produces, making it unavailable for the host cell membranes. This results in membrane fluidity, increased adherence of macrophages to endothelial cells, and subsequently the risk of developing atherogenic lesions. Tocotrienol is expected to inhibit intracellular synthesis of cholesterol, thus reducing cholesterol hijacking and Golgi trafficking of cholesterol by *Chlamydial.* Therefore, tocotrienol is expected to inhibit the progression or development of *Chlamydia* from RB to EB, and concomitantly limits the enlargement process of chlamydial inclusion formation. Finally, this would reduce or retard bacterial replication in infected cells that support subsequent infection of uninfected host cells, which occurs following lysis of *Chlamydia*-infected host cells [Figure 1, steps 4→1]. It also may be possible that lipid raft-mediated chlamydial entry is not required for infection (Gabel *et al.,* 2004). Therefore, tocotrienol may inhibit chlamydial infection in mechanism(s) besides the cholesterol reduction route. Nonetheless, tocotrienol inhibits chlamydial infection. However, the cholesterol reduction route may still account for the inhibition of chlamydial progression.

Therefore, the summary of this invention is that delta tocotrienol inhibits cholesterol *de novo* synthesis, disrupting or inhibiting lipid raft formation, although its involvement in restricting lipid rafts may not be necessary. By this invention, tocotrienol restricts or halts the entry (endocytosis) via pathogen-host adhesion and the progression of the various stages of the chlamydial developmental cycle. Delta tocotrienol may have a dual purpose of simultaneous prevention of infection and arrest of normal chlamydial developmental progression that would lead to formation of new infectious EBs. Therefore, this invention describes that the inhibition of cholesterol in the cholesterol-rich lipid raft impedes the progression of *Chlamydia* infection in all of its developmental stages.

### Definitions

**Assorted Nutritional Supplements** Plant sterols, oryzanols, corosolic acid, policosanols, B vitamins (e.g., pentathine, niacin, carnitine, alpha-lipoic acid, taurine), CoQ10, omega 3 fatty acids (e.g., DHAs, EPAs, alpha linoleic acid), lecithins (e.g., phosphotidyl-choline, serine, ethanolamine, inositol), garlic, gugul lipids, insoluble and soluble fibers, soy protein (e.g., oligo- and poly-peptides, hydrolysates), chitosan, red yeast rice (*Monascus*), and minerals (e.g., magnesium, calcium, chromium, potassium).

**Caveolae** -- Small pockets, vesicles, caves, or recesses communicating with the outside of a cell and expending inward, causing indents in the cytoplasm and cell membrane. Such caveolae may be pinched off to form free vesicles within the cytoplasm. They are considered sites for uptake of materials into the cell, and are one of the routes *Chlamydial* takes to enter host cells. Caveolae are also sites of expulsion of materials from the cell, or sites of addition or removal of cell (unit) membrane to or from the cell surface.

***Chlamydial -*** Bacteria belonging to the order of *Chlamydiales*, and are contained in the family of obligate intracellular organisms, which includes other obligate intracellular bacteria, viruses, and parasites, as well as the different species of *Chlamydial* such as *Chlamydial trachomatis, Chlamydiophila pneumonia, Chlamydiophila pecorum, Chlamydiophila psitacci, Chlamydiophila abortus, Chlamydiophila felis, Chlamydiophila caviae, Chlamydial suis,* and *Chlamydia muridarum*.

***Chlamydia* Infection -** Initial infection of host cells by *Chlamydial.* This occurs with the infectious morphological form of *Chlamydia,* the elementary body (see Figure 1).

***Chlamydial* Progression -** Development of *Chlamydial* within host cells. This involves transformation of the infectious elementary body to the non-infectious reticulate body, transformation back to the infectious elementary body form, and growth of the *Chlamydia*-containing inclusion up to the point of cell lysis (see Figure 1).

***Chlamydia*-Reinfection** - At completion of the chlamydial developmental cycle, the infected host cell undergoes cell lysis, setting free infectious elementary bodies (see Figure 1). These infectious elementary bodies then infect neighboring host cell.

***Chlamydia*-Related Diseases** -- Cardiovascular disease, hypertension, atherosclerosis, COX-1- and COX-II-induced inflammation, sexually transmitted disease and genital tract infection, arthritis, respiratory tract infection and pneumonia, ocular infection, neurological diseases, including Alzheimer's disease and multiple sclerosis.

**Clathrin-Coated Pits** - Involved in internalization of receptor-bound ligands by receptor-mediated endocytosis. This is one of the pathways *Chlamydial* uses to enter a cell.

**Cholesterol-Reducing Drugs** -- Statins (e.g., lovastatin, simvastatin, pravastatin), citrus bioflavonoids, or specifically polymethoxylated flavones (e.g., tangeretin, nobiletin, hesperidin, rutin), polyphenolics (e.g., EGCG, catechins, resveratrol).

**Lipid Rafts -** Domains high in sphingolipids and cholesterol in the cell membrane. These domains are detergent-insoluble glycolipid-rich domains and move within the fluid bilayer. These lipid rafts are one of the pathways by which *Chlamydia* enter host cells.

**Tocochromanol** - Vitamin E. This includes all individual isomers of tocopherol and tocotrienol, tocotrienol-rich fractions from natural sources such as palm, rice, and annatto, and various spectrum vitamin E (e.g., full and appropriate spectra).

### Examples

### Example 1

Chlamydial Strains: Stocks of *C. trachomatis* serovar K/VR887 were grown in J774A.1 cells without centrifuge assistance. Infected cells were lysed, this stock aliquoted and frozen down in SPG freeze medium (75.0 g sucrose, 0.52 g potassium phosphate, 1.22 g sodium phosphate dibasic, 0.72 g glutamic acid, diluted in 100 ml ddH₂O). These aliquots were stored in liquid N₂ or at -80° C, and later thawed for use to infect monolayers.

Cell Lines Used: Mouse macrophages (J774A.1), human mammary tumor cells (MCF-7), and human epithelial cells (Hep-2), were obtained from the American Type Culture collection. Human mammary tumor cells (TMX2-28) were a kind gift from Dr. Arcaro, and human B-lymphocytes (JY) were a kind gift from Dr. Eric Martz. All cell lines were maintained in Richter's improved MEM insulin (IMEMZO, Irvine Scientific, Santa Ana, CA) with 5% fetal bovine serum (FBS, Atlanta Biologicals, Norcross, GA). Cells were grown to 80% confluence on 12 mm coverslips in 12 well plates (Becton Dickinson Labware, Franklin Lakes, NJ). A dilution of 1:125 or 1:200 of the *C. trachomatis* serovar K stock was made using the standard complete cycloheximide overlay media (Bio-Whittaker, Walkersville, Md.) containing 10% FBS, and 1X L-glutamine (CCOM). This was layered onto the coverslip containing monolayers, and incubated for 24-48 hours at 37° C with 5% CO₂. Coverslips with the cell monolayers were harvested, rinsed with phosphate buffered saline (PBS), fixed with 70% cold methanol, stored and subsequently immunostained.

Delta Tocotrienol Treatment: A stock of delta tocotrienol (98% purity, American River Nutrition, Hadley, MA) was diluted to 1 mg/1 ml in absolute ethanol [EtOH]. Confluent monolayers of J774A.1, MCF-7, TMX2-28, JY, or Hep-2 cells were treated with 5, 10,20, 30, or 40 µmol/L concentrations of delta tocotrienol. Treatment occurred 5 hours prior to infection with *C. trachomatis* serovar K, at point of infection, or three hours post-infection.

Immunostaining: Briefly, infected cells were immunostained with a 1:2 dilution of guinea pig anti-chlamydia polyclonal antibody (Biomeda, Foster City, CA), or a 1:125 dilution of rabbit anti-chlamydia EB whole serum for 1 hour at 37° C. Following PBS rinses X3, the bound antibodies were detected using a 1:100 dilution of FITC-conjugated donkey anti-guinea pig or a 1:125 dilution of TRITC-conjugated goat anti-rabbit secondary antibodies (Jackson ImmunoResearch, West Grove, PA). Following incubation for 1 hour at room temperature and 3 rinses with PBS, coverslips were mounted onto slides using Fluoromount-G (Southern Biotechnology Associates Inc., Birmingham, AL) or Vectashield® Mounting Medium with DAPI (Vector Laboratories, Inc., Burlingame, CA), and were then sealed. Initially, slides were examined at 400X using a Nikon LABPHOT-2. For photography, slides were assessed in the Massachusetts Central Microscopy Facility using a Zeiss LSM 510 Meta Confocal System and 630X magnification. Images were captured, and as relevant, merged using the Confocal Assistant™ version 4.02 Image Processing Software.

At this magnification, the differences in the number of inclusions present in the tocotrienol-treated sample (Figure 3A) and control (Figure 3B) were readily visualized. As the white arrows point out, no evidence of large, mature inclusions was found with tocotrienol-treatment, whereas untreated cells (3B) exemplify a high level of infection by *Chlamydia*, as shown by the large number and size of the inclusions present.

Quantitatively, immunofluorescence staining showed that the number of chlamydial inclusions was decreased significantly in tocotrienol-treated cells (Figure 3, Table 1). This showed that the initial infection by chlamydial EBs of host cells was impeded and suppressed (Figure 1, step 1).

### Example 2

The methods of Example 1 were used in this study. Cells in culture were treated with delta tocotrienol concentrations of 5, 10, 20, 30, and 40 µmol/L, where the viscous Vitamin E compound is diluted in 100% EtOH without cytotoxicity. Controls incubated with the corresponding amounts of EtOH, as well as controls incubated with delta tocotrienol diluted in EtOH showed no difference in cell number when compared to cells grown in culture medium. At 630X magnification, the difference in size and morphology of inclusions was seen when comparing the tocotrienol-treated sample (Figure 4A) to the control (Figure 4B). Inclusions in (4A) were small, and did not fuse to the morphology of mature inclusions. In (4B), inclusions were mature, large, and solidly stained. Optical sections through the Z-axis (third dimension) demonstrate that these untreated cells (4B) were three-fold thicker than the tocotrienol-treated cells (4A). Therefore, the chlamydial inclusion volumes would be expected to be even larger (see Example 3).

Chlamydial inclusions in cells that were treated with higher concentrations (20-40 µmol/L) of delta tocotrienol were immature, small, and less round when visualized by *Chlamydia*-specific immunofluorescence staining (Figure 4). This observation showed that chlamydial development within the host cell was grossly inhibited (Figure 1, step 2→3).

### Example 3

**Table 1. Summary of Inclusion and Mouse Macrophage Cell Count**

| Inclusions (per field of view) | First Infection | | Second Infection | | Third Infection | |
|---|---|---|---|---|---|---|
| | Treated | Control | Treated | Control | Treated | Control |
| Large (15-20 µm) | 0.7 | 2.6 | 1.4 | 2.6 | 0.7 | 1.2 |
| Small (≤10 µm) | 4.5 | 10.0 | 0.6 | 3.2 | 1.1 | 2.1 |
| Total | 5.2 | 12.6 | 2.0 | 5.8 | 1.8 | 3.3 |
| Total/Cell | 0.058 | 0.111 | 0.032 | 0.073 | 0.016 | 0.041 |
| Percent Inhibition | 52.3% | | 43.8% | | 39.0% | |

| | | | | | | |
|---|---|---|---|---|---|---|
| * Controls were cells that were infected, but never treated with delta tocotrienol | | | | | | |

The methods of Example 1 were used in this study. Counts on all coverslips with experimental conditions as in Figure 3 and 4 were done for the average of 10 fields of view. Cells were treated with 30 µmol/L concentrations of delta tocotrienol. Re-infectability was studied, where transfer of the supernatant containing infectious elementary bodies (EBs) from infected, tocotrienol-treated cells to uninfected, tocotrienol-treated cells (Table 1).

This observation suggested that repeated use of delta tocotrienol significantly reduces the level of infection by *Chlamydia*, and showed that delta tocotrienol inhibited chlamydial EBs from re-infecting host cells (Figure 1, step 4→1). Therefore, tocotrienol helped the host cells to resist the repeated attempts of infection by *Chlamydial.*

The percent inhibition of inclusion formation in tocotrienol-treated as compared to untreated cells was approximately 40-50% (Table 1). It also should be noted that large inclusions of the control cells were larger (20 µm; more like Figure 1, step 2→3) than large inclusions in the tocotrienol-treated cells (10-15 µm; more like Figure 1, step 1→2). For example, tocotrienol-treated chlamydial inclusions were typically 2 to 3-fold smaller (X-Y axes) and 3-fold thinner (Z-axis; Example 2) than inclusions in control cells. The chlamydial inclusion volume thus calculated (2x3 to 3x3) was 6-9-fold smaller; suggesting that tocotrienol effectively inhibited the normal chlamydial cell cycle (Figure 1, steps 1→2 and 2→3).

The overall effect of delta tocotrienol on *Chlamydia*-infected cells was that it reduced infection by influencing the pathogen's infectivity and by inhibiting its normal development (Table1, Figures 3 and 4), thus compromising the pathogen's entire developmental cycle (Figure 1). This study showed that repeated doses of tocotrienol combat *Chlamydia* infection and progression.

It is likely that control and eradication of *Chlamydial* in humans will require tocotrienol supplementation for an extended period. In light of this, tocotrienols and other agents (such as tangeretin, nobiletin, EGCG, and resveratol; more in Example 7) in monotherapies or in combination are superior because of their lack of toxicity. In contrast, other drugs (such as statins) have sustained toxicities with chronic usage. This invention highlights the use of safe natural products for fighting infections.

### Example 4

The methods of Example 1 were used in this study. Samples were analyzed for carriage of *Chlanydia*-infected cells by FACScan (Becton, Dickinson and Company, Franklin Lakes, NJ).

For each period, infection was decreased with delta-tocotrienol treatment Chlamydial inhibition in JY cells was at least 2-fold over an infection period of 72 hours, with a 2.6-fold maximum inhibition at 36 hours (Figure 5).

Clearly, chlamydial inhibition was effective in as short a period as 1 day, and following one single tocotrienol treatment, the effect was persistent for at least 3 days. This study provides showed that at least one-time dose of tocotrienol is effective against *Chlamydial.*

### Example 5

Flow Cytometry Assessment of WBC: Flow cytometry (FC) quantification of *Chlamydia*-infected peripheral WBC used PBS rinsed buffy coat (BC) from HP samples. The BC cells were fixed and permeabilized (1% paraformaldehyde and 1% Triton X-100, 10 min. at RT; Aldrich Chemical Company, Inc., Milwaukee, WI). BCs were separately incubated for 1 hour with a 1:200 dilution of rabbit anti-*Chlamydia* primary antibody (Biodesign International, Saco, ME) followed by a 1 hour incubation with a 1:150 dilution of FITC-conjugated goat anti-rabbit IgG (H+L) (Jackson ImmunoResearch, West Grove, PA), PBS rinsed X3, and mono-dispersed by passage through a nylon mesh filter (Lab-Line Instruments Inc, Melrose Park, IL). Sufficient WBCs were added to obtain 10,000 cells/tube and samples were analyzed for carriage of *Chlamydia*-infected cells by FACScan (Becton, Dickinson and Company, Franklin Lakes, NJ).

In this patient (Figure 6), tocotrienol consumption reduced the granularity and amount of *Chlamydia*-infected cells, which is represented by the *Chlamydia*-negative population in the lower left quadrant of the panel.

Since flow cytometry samples a larger cell population [-10,000 cells], this human study further supported and corroborated the chlamydial inhibition observed in the microscopic studies of *Chlamydia* infected cells treated *in vitro* (Figures 3 and 4) and shown in the earlier four examples.

This study shows that tocotrienol is effective in humans against *Chlamydia* as represented in circulating WBCs, including neutrophils, monocytes, lymphocytes, eosinophils, and basophils.

### Example 6

**Table 2. Comparison of LDL Levels and Chlamydial Infection Status in Hyperlipidemic Patients with Delta Tocotrienol Supplementation**

| | Patient 1 | Patient 2 | Patient 3 |
|---|---|---|---|
| % Decrease in LDL | 20% | 7% | 25% |
| Chlamydial Infection Status before Delta-Tocotrienol Supplementation | negative | positive | strong positive |
| Chlamydial Infection Status after Delta-Tocotrienol Supplementation | negative | negative | negative |

Detection of *Chlamydia*-Infected White Blood Cells (WBC): Smears of whole blood from each10 ml hyperlipidemic HP blood sample, average donor age 54 years, were fixed with 70% MEOH for subsequent staining. Immunostaining used a 1:125 dilution of rabbit polyclonal anti-*Chlamydia* EB antibody followed by a 1:125 dilution of TRITC-conjugated goat anti-rabbit antibody (H+L) (Jackson ImmunoResearch, West Grove, PA). Slides were incubated at room temperature for 1 hour with each diluted antibody, rinsed, mounted and sealed as described above. Digital images of optical sections through these samples were acquired with a Zeiss LSM 510 Meta Confocal System.

In a clinical study, buffy coats from blood samples drawn both before and after delta-tocotrienol supplementation were immunostained for detection of *Chlamydia*-infected cells. The samples were tested for LDL levels both before and after delta-tocotrienol supplementation.

Tocotrienol lowered cholesterol in patients with or without prior chlamydial infection. Apparently, a cholesterol drop was more significant when the hyperlipidemic patient was *Chlamydia*-negative (Patient 1 vs. 2). As discussed earlier (Sawayama *et al.,* 2003), pravastatin only lowered cholesterol marginally in *Chlamydia*-positive patients albeit that the carotid plaques were significantly reduced. This is consistent with the four-year clinical study described earlier, wherein patients who took tocotrienols had progressive carotid arteriosclerosis regression, but their cholesterol did not drop until the fourth year (Kooyenga *et al.,* 2001).

However, in patient 2, the chlamydial infection status reversed from positive to negative with tocotrienol supplementation. When chlamydial inhibition responded to tocotrienol supplementation strongly (Patient 3), the cholesterol drop was also larger, similar to the patient that was *Chlamydia*-negative. Since tocotrienol is known to lower cholesterol, such cholesterol synthesis restriction inhibited the cholesterol-requiring growth of *Chlamydia* (Carabeo, 2003), seen in patients 2 and 3.

Tocotrienol supplementation reduced the cholesterol level and chlamydial infection in hyperlipidemic patients, and rendered the chlamydial infection status negative.

### Example 7

A corollary of example 6 is that any agent that restricts cholesterol synthesis would therefore prevent or retard *Chlamydia* from hijacking cholesterol. Such agents include statins (e.g., lovastatin, simvastatin, pravastatin), citrus bioflavonoids, or specifically polymethoxylated flavones (e.g., tangeretin, nobiletin, hesperidin, rutin; English, 2004), polyphenolics (e.g., EGCG, catechins, resveratrol; Yamazaki, 2005), and an assortment of nutritional supplements (e.g., plant sterols, B vitamins, omega 3 fatty acids, insoluble and soluble fibers, red yeast rice; Strum, Faloon, 2005). This list is not meant to be limiting, but to exemplify the effect of cholesterol-lowering agents on restriction of *Chlamydia* infection. Other non-limiting examples for cholesterol-lowering, lipid-lowering, and cardiovascular support agents have recently been widely published (English, 2005; Granato, 2003 & 2005; Myers, 2006; Naguib, 2004; Srejic, 2006).

This study shows that any agent that restricts cholesterol/lipids also restricts *Chlamydial* infection/growth. In other words, any agent that restricts cholesterol restricts *Chlamydia*.

### Example 8

The methods of Example 1 were used in this study. Untreated cancer cells showed evidence of solid large inclusion (>20 µm; Figure 7B) compared to intermediate size inclusions (20 µm) in non-cancerous murine macrophages (Figure 4B) and even smaller inclusions in tocotrienol treated cells, both murine macrophages (Figure 4A) and mammary tumor cells (10-15 µm; Figure 7A).

The cholesterol content of cancer cells is very high because the rate-limiting HMG-CoA reductase enzyme for cholesterol synthesis in these cells is aberrantly elevated and the enzyme is resistant to sterol feedback regulation. Unequivocally, tocotrienol has been shown to inhibit the reductase synthesis and accelerate reductase degradation (Mo and Elson, 2004).

Since cancer cells are high in cholesterol content, they are more susceptible to infection by cholesterol-hijacking *Chlamydia*, which explains the engorged inclusions within the cells.

Tocotrienols are known to induce apoptosis of cancer cells. Contrarian to this, *Chlamydia* has been shown to inhibit the apoptosis of infected cells. *Chlamydia*-infected, tocotrienol-treated cancer cells in this example did not appear to become apoptotic. Although tocotrienol did not cause infected host cells to undergo apoptosis, it nonetheless disrupted the development of full-blown inclusions by the infecting *Chlamydia.* However, apoptosis with tocotrienol is expected with continual usage (Example 3), whereby the chlamydial EBs are eliminated with time (Example 1), as it has been numerously shown that control of *Chlamydia* is a process of controlling its progression (all earlier examples). This study shows tocotrienol will arrest opportunistic chlamydial infections in cancer patients.

### Example 9

Chlamydial infection modulated activation of T-lymphocytes and was linked to a decrease in the antigen-presenting dendritic cell population in the human body. Therefore, the dendritic cells are prevented from activating the T-lymphocytes, and the immunity of the individual against *Chlamydia* and other opportunistic pathogens is decreased. This is further supported by the implication of *Chlamydial* in autoimmune disease. Tocotrienol is known to boost the immune system to ward off viruses and bacterial infections, and has even been shown to assist T-lymphocytes in slowing down the progression of AIDS and increasing specific immune markers. Thus, tocotrienol also aids the immune system through the activation of dendritic cells and T-lymphocytes to fight off chlamydial infections.

This study shows tocotrienol will ward off pathogenic infections including *Chlamydial* via activation of antigen-presenting dendritic cells and T-lymphocytes.

This invention also applies to activation of antigen-presenting dendritic cells and T-lymphocytes against tumors and cancers.

### Example 10

Tocotrienol decreases high blood pressure in hypertensive rats. Primary pulmonary hypertensive (PPH) cells have enhanced expression of caveolae, which contributes to the elevated [Ca²⁺] associated with hypertension, and when treated with cholesterol-reducing agents such as statin, caveolae expression in these cells was modified and vasoconstriction was reduced. Since PPH cells have increased caveolae expression in cell membranes and *Chlamydial* entry into host cells involves lipid rafts, hypertensive patients are more susceptible to opportunistic chlamydial infections. Tocotrienol, like other cholesterol-reducing agents, decreases caveolae expression in cell membranes, downregulates the [a²⁺] in PPH cells, and protects against chlamydial infections.

This invention provides the application that tocotrienol fights off opportunistic chlamydial infection of hypertensive patients, and thereby reduces their blood pressure.

### Example 11

Metabolic syndrome is a cluster of cardiovascular risk factors, including elevated waist circumference, elevated triglycerides, reduced high-density lipoprotein cholesterol, elevated blood pressure, and elevated fasting glucose associated with obesity, and elevates one's risk of developing diabetes. *Chlamydial* is not a causative agent of diabetes, but seroactive chlamydial infections in diabetic patients are more frequent than in non-diabetic patients, meaning that diabetic patients may be more susceptible to infection by *Chlamydia.* Tocotrienol reduces the cardiovascular risk factor associated with metabolic syndrome, obesity, and diabetes, and therefore may reduce opportunistic chlamydial infections due to these factors.

This invention provides the application that tocotrienol reduces cardiovascular risk factors associated with metabolic syndrome, and thereby reduces the risk of diabetes and concomitant or subsequent infection by *Chlamydial.*

### Example 12

The mode of application of tocotrienols is important because *Chlamydial* is involved in many public health-related diseases. A non-limiting summary of these diseases is described in the background section. The mode of application of tocotrienol may be in form of aerosol sprays (to reach respiratory tract airways), oral ingestion via softgels, tablets or capsules (to reach vascular-organistic systems), topical creams, douches, and lotions (to reach genital sites), and topical liquid drops (to reach ocular sites).

Dosages: In one embodiment, the invention is drawn to a method comprising administering delta-T3 in a range from 10 to 1000 mg per day. In a preferred embodiment, the invention is drawn to a method comprising administering delta-T3 in a range from 20 to 500 mg per day. In a more preferred embodiment, the invention is drawn to a method comprising administering delta-T3 in a range from 50 to 150 mg per day. Treatment would be continuous with the delta-T3 being administered daily at the above-mentioned dosages (for as short as one month, preferably six months, and most preferably one year), or until infection and inflammation due to *Chlamydia* is cleared (e.g., no inclusion-forming units can be found in the patient's whole blood). However, for *prevention* of chlamydial infection the dosage can be on the lower end (e.g., 1-100 mg/day), and the dosage duration can be indefinite, and begun before the subject has any evidence of infection by *Chlamydial.*

To reach respiratory tract infections, tocotrienol may be used in form of aerosol spray, at a dosage of 1-4 sprays per day at the above-mentioned dosages. For genital tract infections, tocotrienol may be administered in form of topical creams, lotions, or douches with 1-4 applications per day at the above-mentioned dosages. Vascular-organistic systems inflamed due to chlamydial infection can be treated with oral ingestion of tocotrienol with 1-4 softgels, tablets, or capsules per at the above-mentioned dosages. To treat ocular infections, including conjunctivitis, tocotrienol may be administered in form of liquid eye drops with 1-4 applications per day at the above-mentioned dosages.

## Claims

1. A tocotrienol for use in the treatment of a *Chlamydia* infection, wherein said tocotrienol is administered for at least 3 days to a mammal in need of treatment.

2. Use of a tocotrienol for the preparation of a medicament for the treatment of a *Chlamydia* infection , wherein said medicament is administered for at least 3 days to a mammal in need of treatment.

3. The tocotrienol of claim 1 or the use of claim 2, wherein the treatment inhibits, prevents or disrupts the developmental cell cycle and infection of *Chlamydia.*

4. The tocotrienol of claim 1 or 3 or the use of claim 2 or 3, wherein the treatment further comprises administering a geranyl geraniol.

5. The tocotrienol of any one of claims 1 or 3 to 4 or the use of any one of claims 2 to 4, wherein said tocotrienol is selected from the group consisting of a natural tocotrienol and a synthetic tocotrienol.

6. The tocotrienol or the use of claim 5, wherein said tocotrienol is selected from the group consisting of alpha-tocotrienol, beta-tocotrienol, gamma-tocotrienol, delta-tocotrienol, desmethyl-tocotrienol, and didesmethyl-tocotrienol.

7. The tocotrienol or the use of claim 6, wherein said tocotrienol is delta-tocotrienol.

8. The tocotrienol of any one of claims 1 or 3 to 7 or the use of any one of claims 2 to 7, wherein the *Chlamydia* is selected from the group consisting of *Chlamydia trachomatis, Chlamydia suis, Chlamydia muridarum, Chlamydiophila pneumoniae, Chlamydiophila psittaci, Chlamydiophila pecorum, Chlamydiophila abortis, Chlamydiophila felis,* and *Chlamydiophila caviae.*

9. The tocotrienol of any one of claims 1 or 3 to 8 or the use of any one of claims 2 to 8, wherein the tocotrienol stunts the growth and inhibits *Chlamydia* maturation of reticulate bodies into elementary bodies, preventing its progression and development.

10. The tocotrienol of any one of claims 1 or 3 to 9 or the use of any one of claims 2 to 9, wherein the tocotrienol reduces chlamydial infection of a white blood cell.

11. The tocotrienol or the use of claim 10, wherein the white blood cell is selected from the group consisting of neutrophils, monocytes, lymphocytes, eosinophils, and basophils.

12. The tocotrienol of any one of claims 1 or 3 to 11 or the use of any one of claims 2 to 11, wherein the mode of application, of the tocotrienol is selected from the group consisting of aerosol spray, oral ingestion, creams, douches, lotions, and eye drops.

13. The tocotrienol or the use of claim 12, comprising administering a dose of tocotrienol between 20 mg and 500 mg per day, or between 50 ing and 150 mg per day.

14. A tocotrienol for use in the treatment of a *Chlamydia* infection, wherein said tocotrienol is administered in combination with at least one agent selected from the group consisting of a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.

15. Use of tocotrienol for the preparation of a medicament for the treatment of a *Chlamydia* infection, wherein said tocotrienol is administered in combination with at least one agent selected from the group consisting of a statin, a bioflavonoid, a polyphenolic, a polymethoxylated flavone, a plant sterol, a oryzanol, a policosanol, a B vitamin, CoQ10, an omega 3 fatty acid, a lecithin, garlic, a gugul lipids, an insoluble fiber, a soluble fiber, a soy protein, a chitosan, a red yeast rice, and a mineral.

16. The tocotrienol of claim 14 or the use of claim 15, wherein the agent is citrus bioflavonoid.

17. A tocotrienol for use in the treatment of a *Chlamydia* infection, wherein tocotrienol is administered for at least 3 days to a mammal or avian, wherein said mammalian or avian suffers from a *Chlamydia*-associated disease selected from the group consisting of cardiovascular disease, hypertension, atherosclerosis, COX-I- and COX-II-induced inflammation, a sexually transmitted disease, a genital tract infection, arthritis, prediabetes, metabolic syndrome, diabetes, polycystic ovarian syndrome (PCOS), a respiratory tract infection, pneumonia, an ocular infection, a neurological disease, Alzheimer's disease, and multiple sclerosis.

18. Use of tocotrienol for the preparation of a medicament for the treatment of a *Chlamydia* infection, wherein tocotrienol is administered for at least 3 days to a mammal or avian, wherein said mammalian or avian suffers from a *Chlamydia-*associated disease selected from the group consisting of cardiovascular disease, hypertension, atherosclerosis, COX-I- and COX-II-induced inflammation, a sexually transmitted disease, a genital tract infection, arthritis, prediabetes, metabolic syndrome, diabetes, polycystic ovarian syndrome (PCOS), a respiratory tract infection, pneumonia, an ocular infection, a neurological disease, Alzheimer's disease, and multiple sclerosis.

19. A tocotrienol for use in improving immunity to *Chlamydia,* wherein said tocotrienol potentiates an antigen-presenting dendritic cell.

20. Use of tocotrienol for the manufacture of a medicament for improving immunity to *Chlamydia,* wherein said tocotrienol potentiates an antigen-presenting dendritic cell.

## Patentansprüche

1. Tocotrienol zur Verwendung in der Behandlung einer *Chlamydia*-Infektion, wobei das Tocotrienol für mindestens 3 Tage einem Säuger verabreicht wird, der einer Behandlung bedarf.

2. Verwendung eines Tocotrienols zur Herstellung eines Medikaments zur Behandlung einer *Chlamydia*-Infektion, wobei das Medikament für mindestens 3 Tage einem Säuger verabreicht wird, der einer Behandlung bedarf.

3. Tocotrienol nach Anspruch 1 oder die Verwendung nach Anspruch 2, wobei die Behandlung den Entwicklungszellzyklus und die Infektion von *Chlamydia* inhibiert, vorbeugt oder unterbricht.

4. Tocotrienol nach Anspruch 1 oder 3 oder die Verwendung nach Anspruch 2 oder 3, wobei die Behandlung zudem die Verabreichung eines Geranylgeraniols umfasst.

5. Tocotrienol nach einem der Ansprüche 1 oder 3 bis 4 oder die Verwendung nach einem der Ansprüche 2 bis 4, wobei das Tocotrienol ausgewählt ist aus der Gruppe bestehend aus einem natürlichen Tocotrienol und einem synthetischen Tocotrienol.

6. Tocotrienol oder die Verwendung nach Anspruch 5, wobei das Tocotrienol ausgewählt ist aus der Gruppe bestehend aus Alpha-Tocotrienol, Beta-Tocotrienol, Gamma-Tocotrienol, Delta-Tocotrienol, Desmethyl-Tocotrienol und Didesmethyl-Tocotrienol.

7. Tocotrienol oder die Verwendung nach Anspruch 6, wobei das Tocotrienol Delta-Tocotrienol ist.

8. Tocotrienol nach einem der Ansprüche 1 oder 3 bis 7, oder die Verwendung nach einem der Ansprüche 2 bis 7, wobei die *Chlamydien* ausgewählt sind aus der Gruppe bestehend aus *Chlamydia trachomatis, Chlamydia suis, Chlamydia muridarum, Chlamydiophila pneumoniae, Chlamydiophila psittaci, Chlamydiophila pecorum, Chlamydiophila abortis, Chlamydiophila felis* und *Chlamydiophila caviae.*

9. Tocotrienol nach einem der Ansprüche 1 oder 3 bis 8, oder die Verwendung nach einem der Ansprüche 2 bis 8, wobei das Tocotrienol das Wachstum hemmt und die Reifung von Retikularkörperchen zu Elementarkörperchen von *Chlamydia* inhibiert, so dass deren Verlauf und Entwicklung verhindert wird.

10. Tocotrienol nach einem der Ansprüche 1 oder 3 bis 9, oder die Verwendung nach einem der Ansprüche 2 bis 9, wobei das Tocotrienol die Chlamydieninfektion eines weißen Blutkörperchens reduziert.

11. Tocotrienol oder die Verwendung nach Anspruch 10, wobei das weiße Blutkörperchen ausgewählt ist aus der Gruppe bestehend aus Neutrophilen, Monocyten, Lymphozyten, Eosinophilen und Basophilen.

12. Tocotrienol nach einem der Ansprüche 1 oder 3 bis 11, oder die Verwendung nach einem der Ansprüche 2 bis 11, wobei die Darreichungsform des Tocotrienols ausgewählt ist aus der Gruppe bestehend aus Aerosolspray, oraler Aufnahme, Cremes, Spülungen, Lotionen und Augentropfen.

13. Tocotrienol oder die Verwendung nach Anspruch 12, das die Verabreichung einer Dosis an Tocotrienol zwischen 20 mg und 500 mg pro Tag, oder zwischen 50 mg und 150 mg pro Tag umfasst.

14. Tocotrienol zur Verwendung in der Behandlung einer *Chlamydia*-Infektion, wobei das Tocotrienol in Kombination mit mindestens einem Agens verabreicht wird, das ausgewählt ist aus der Gruppe bestehend aus einem Statin, einem Bioflavonoid, einem Polyphenol, einem polymethoxylierten Flavon, einem pflanzlichen Sterol, einem Oryzanol, einem Policosanol, einem B-Vitamin, CoQ10, einer Omega-3-Fettsäure, einem Lecithin, Knoblauch, einem Guggullipid, einem unlöslichen Ballaststoff, einem löslichen Ballaststoff, einem Sojaprotein, einem Chitosan, einem roten Hefereis und einem Mineral.

15. Verwendung von Tocotrienol zur Herstellung eines Medikaments zur Behandlung einer *Chlamydia*-Infektion, wobei das Tocotrienol in Kombination mit mindestens einem Agens verabreicht wird, das ausgewählt ist aus der Gruppe bestehend aus einem Statin, einem Bioflavonoid, einem Polyphenol, einem polymethoxylierten Flavon, einem pflanzlichen Sterol, einem Oryzanol, einem Policosanol, einem B-Vitamin, CoQ10, einer Omega-3-Fettsäure, einem Lecithin, Knoblauch, einem Guggullipid, einem unlöslichen Ballaststoff, einem löslichen Ballaststoff, einem Sojaprotein, einem Chitosan, einem roten Hefereis und einem Mineral.

16. Tocotrienol nach Anspruch 14 oder die Verwendung nach Anspruch 15, wobei das Agens Zitrusbioflavonoid ist.

17. Tocotrienol zur Verwendung in der Behandlung einer *Chlamydia*-Infektion, wobei Tocotrienol für mindestens 3 Tage einem Säuger oder einem Vogel verabreicht wird, wobei der Säuger oder der Vogel an einer *Chlamydia-*begleitenden Krankheit leidet, die ausgewählt ist aus der Gruppe bestehend aus kardiovaskulärer Erkrankung, Bluthochdruck, Arteriosklerose, COX-I- und COX-II-induzierter Entzündung, einer sexuell übertragbaren Krankheit, einer Infektion des Genitaltrakts, Arthritis, Prädiabetes, metabolisches Syndrom, Diabetes, polyzystisches Ovariensyndrom (polycystic ovarian syndrome; PCOS), einer Infektion des Atemtrakts, Lungenentzündung, einer Augenentzündung, einer neurologischen Erkrankung, Alzheimer'sche Krankheit und multipler Sklerose.

18. Verwendung von Tocotrienol zur Herstellung eines Medikaments zur Behandlung einer *Chlamydia*-Infektion, wobei Tocotrienol für mindestens 3 Tage einem Säuger oder einem Vogel verabreicht wird, wobei der Säuger oder der Vogel an einer *Chlamydia*-begleitenden Krankheit leidet, die ausgewählt ist aus der Gruppe bestehend aus kardiovaskulärer Erkrankung, Bluthochdruck, Arteriosklerose, COX-I- und COX-II-induzierter Entzündung, einer sexuell übertragbaren Krankheit, einer Infektion des Genitaltrakts, Arthritis, Prädiabetes, metabolisches Syndrom, Diabetes, polyzystisches Ovariensyndrom (polycystic ovarian syndrome; PCOS), einer Infektion des Atemtrakts, Lungenentzündung, einer Augenentzündung, einer neurologischen Erkrankung, Alzheimer'sche Krankheit und multipler Sklerose.

19. Tocotrienol zur Verwendung in der Verbesserung der Immunität gegen *Chlamydia,* wobei das Tocotrienol eine Antigen-präsentierende dendritische Zelle verstärkt.

20. Verwendung von Tocotrienol zur Herstellung eines Medikaments zur Verbesserung der Immunität gegen *Chlamydia,* wobei das Tocotrienol eine Antigen-präsentierende dendritische Zelle verstärkt.

## Revendications

1. Tocotriénol destiné à être utilisé dans le traitement d'une infection par un *Chlamydia,* dans lequel ledit tocotriénol est administré pendant 3 jours au moins à un mammifère qui nécessite un traitement.

2. Utilisation d'un tocotriénol pour la préparation d'un médicament destiné au traitement d'une infection par *Chlamydia*, ledit médicament étant administré pendant 3 jours au moins à un mammifère qui nécessite un traitement.

3. Tocotriénol selon la revendication 1 ou utilisation selon la revendication 2, le traitement freinant, empêchant ou perturbant le cycle de développement cellulaire et l'infection par *Chlamydia*.

4. Tocotriénol selon la revendication 1 ou la revendication 3 ou utilisation selon la revendication 2 ou la revendication 3, le traitement comprenant en outre l'administration d'un géraniol de géranyle.

5. Tocotriénol selon l'une quelconque des revendications 1 ou 3 à 4 ou utilisation selon l'une quelconque des revendications 2 à 4, ledit tocotriénol étant sélectionné dans le groupe constitué par un tocotriénol naturel et un tocotriénol synthétique.

6. Tocotriénol ou utilisation selon la revendication 5, ledit tocotriénol étant sélectionné dans le groupe constitué par un alpha-tocotriénol, un bêta-tocotriénol, un gamma-tocotriénol, un delta-tocotriénol, un desméthyle-tocotriénol, et un didesméthyle-tocotriénol.

7. Tocotriénol ou utilisation selon la revendication 6, ledit tocotriénol étant un delta-tocotriénol.

8. Tocotriénol selon l'une quelconque des revendications 1 ou 3 à 7 ou utilisation selon l'une quelconque des revendications 2 à 7, le *Chlamydia* étant sélectionné dans le groupe constitué par *Chlamydia trachomatis, Chlamydia suis, Chlamydia muridarum, Chlamydiophila pneumoniae, Chlamydiophila psittaci, Chlamydiophila pecorum, Chlamydiophila abortis, Chlamydiophila felis,* et *Chlamydiophila caviae.*

9. Tocotriénol selon l'une quelconque des revendications 1 ou 3 à 8 ou utilisation selon l'une quelconque des revendications 2 à 8, le tocotriénol retardant la croissance et empêchant la maturation de *Chlamydia* de corps réticulé en corps élémentaire, en empêchant sa progression et son développement.

10. Tocotriénol selon l'une quelconque des revendications 1 ou 3 à 9 ou utilisation selon l'une quelconque des revendications 2 à 9, le tocotriénol réduisant une infection par *Chlamydia* d'un globule blanc.

11. Tocotriénol ou utilisation selon la revendication 10, le globule blanc étant sélectionné dans le groupe constitué par des neutrophiles, des monocytes, des lymphocytes, des éosinophiles et des basophiles.

12. Tocotriénol selon l'une quelconque des revendications 1 ou 3 à 11 ou utilisation selon l'une quelconque des revendications 2 à 11, le mode d'application du tocotriénol étant sélectionné dans le groupe constitué par un jet d'aérosol, une ingestion orale, des crèmes, des douches, des lotions et des gouttes ophtalmiques.

13. Tocotriénol ou utilisation selon la revendication 12, comprenant l'administration d'une dose de tocotriénol comprise entre 20 mg et 500 mg par jour, ou entre 50 mg et 150 mg par jour.

14. Tocotriénol destiné à être utilisé dans le traitement d'une infection par *Chlamydia,* ledit tocotriénol étant administré en association avec un agent au moins sélectionné dans le groupe constitué par une statine, un bioflavonoïde, un polyphénol, une flavone polyméthoxylée, un stérol végétal, un oryzanol, un policosanol, une vitamine B, un CoQ10, un acide gras Omega 3, une lécithine, de l'ail, des lipides de guggul, une fibre insoluble, une fibre soluble, une protéine de soja, du chitosane, du riz de levure rouge, et un mineral.

15. Utilisation d'un tocotriénol pour la préparation d'un médicament destiné à être utilisé dans le traitement d'une infection par *Chlamydia,* ledit tocotriénol étant administré en association avec un agent au moins sélectionné dans le groupe constitué par une statine, un bioflavonoïde, un polyphénol, une flavone polyméthoxylée, un stérol végétal, un oryzanol, un policosanol, une vitamine B, un CoQ10, un acide gras Omega 3, une lécithine, de l'ail, des lipides de guggul, une fibre insoluble, une fibre soluble, une protéine de soja, du chitosane, du riz de levure rouge, et un mineral.

16. Tocotriénol selon la revendication 14 ou utilisation selon la revendication 15, l'agent étant du bioflavonoïde de citron.

17. Tocotriénol destiné à être utilisé dans le traitement d'une infection par *Chlamydia*, le tocotriénol étant administré pendant 3 jours au moins à un mammifère ou à un oiseau, ledit mammifère ou ledit oiseau souffrant d'une maladie associée à un *Chlamydia* sélectionnée dans le groupe constitué par une maladie cardiovasculaire, une hypertension, une athérosclérose, une inflammation induite par la COX-I et par la COX-II, une maladie sexuellement transmise, une infection des voies génitales, une arthrite, un prédiabète, un syndrome métabolique, un diabète, un syndrome des ovaires polykystiques (PCOS), une infection des voies respiratoires, une pneumonie, une infection oculaire, une maladie neurologique, une maladie d'Alzheimer, et une sclérose en plaques.

18. Utilisation d'un tocotriénol pour la préparation d'un médicament destiné à être utilisé dans le traitement d'une infection par *Chlamydia,* le tocotriénol étant administré pendant 3 jours au moins à un mammifère ou à un oiseau, ledit mammifère ou ledit oiseau souffrant d'une maladie associée à *Chlamydia* sélectionnée dans le groupe constitué par une maladie cardiovasculaire, une hypertension, une athérosclérose, une inflammation induite par la COX-I et par la COX-II, une maladie sexuellement transmise, une infection des voies génitales, une arthrite, un prédiabète, un syndrome métabolique, un diabète, un syndrome des ovaires polykystiques (PCOS), une infection des voies respiratoires, une pneumonie, une infection oculaire, une maladie neurologique, une maladie d'Alzheimer et une sclérose en plaques.

19. Tocotriénol destiné à être utilisé pour renforcer l'immunité vis-à-vis d'un *Chlamydia,* ledit tocotriénol potentialisant une cellule dendritique qui présente un antigène.

20. Utilisation d'un tocotriénol destiné à la fabrication d'un médicament destiné à renforcer l'immunité vis-à-vis de *Chlamydia*, dans lequel ledit tocotriénol renforce une cellule dendritique qui présente un antigène.
